# EUROPÄISCHE PATENTSCHRIFT

(11) **EP 3 668 457 B1**
(45) Veröffentlichungstag und Bekanntmachung des Hinweises auf die Patenterteilung: **07.06.2023**
(21) Anmeldenummer: 17791279.7
(22) Anmeldetag: 17.10.2017
(51) Int. Cl.: A61F 2/28

(54) **VORRICHTUNG ZUR NICHT-INVASIVEN INDUKTION DYNAMISCHER DEFORMATION VON KÖRPERGEWEBE ZUR DIFFERENZIERUNG VON GEWEBEZELLEN**
DEVICE FOR THE NON-INVASIVE INDUCTION OF DYNAMIC DEFORMATION OF BODY TISSUE TO DIFFERENTIATE TISSUE CELLS
DISPOSITIF D'INDUCTION NON INVASIVE DE LA DÉFORMATION DYNAMIQUE DE TISSU CORPOREL POUR LA DIFFÉRENCIATION DE CELLULES TISSULAIRES

(43) Veröffentlichungstag der Anmeldung: 24.06.2020
(73) Patentinhaber: Perren, Nicolas, 4142 Münchenstein (CH)
(72) Erfinder: PERREN, Stephan, 7260 Davos (CH); PERREN, Nicolas, 4142 Münchenstein (CH)
(74) Vertreter: Lusuardi, Werther
(86) Internationale Anmeldenummer: PCT/CH2017/000093
(87) Internationale Veröffentlichungsnummer: WO 2019/075584

(56) Entgegenhaltungen:
- EP-A1- 1 060 731
- DE-A1- 19 600 744
- DE-A1-102006 047 248
- DE-A1-102011 014 789
- US-A- 4 323 056

## Beschreibung

Die Erfindung betrifft eine Vorrichtung zur Erzeugung von Körpergewebedeformation nach dem Oberbegriff des Anspruchs 1.

Aus der DE 10 2011 014789 A1 ist eine Vorrichtung einschließlich einer magnetischen Substanz bekannt.

Es ist bekannt, dass sich reparative Körperzellen unter Deformation in verschiedene Gewebe wie z.B. Knochen umformen. Der Knochen stützt die Elemente des Bewegungsapparats und ermöglicht so dessen Funktion. Durch Überlast gebrochen verliert der Knochen seine mechanische Funktion. Die Reparatur der Funktion des gebrochenen Knochens, die Knochenbruch-Heilung, bedingt eine solide Überbrückung des Bruchs in anatomisch korrekter Lage. Eine erfolgreiche Knochenbruch-Heilung muss induziert, ermöglicht und unterhalten werden. Dabei spielen die mechanischen Bedingungen eine entscheidende Rolle. Dies gilt nicht nur für die Knochenbruch-Heilung sondern auch für Gewebe-Heilung allgemein wie auch für die Herstellung von Geweben im oder ausserhalb Körpers von Mensch und Tier (z.B. tissue engineering).

Verschiedene bekannte medizinische Behandlungsverfahren beruhen auf der Anwendung der Erkenntnisse dieser Dehnungstheorie. So wird zum Beispiel seit Jahren zur Knochenverlängerung oder Defektüberbrückung die Kallusdehnung durch den Ilizarov Apparat, implantierbarer mechatronisch gesteuerter Marknagel und andere ähnlichen Verfahren eingesetzt. Diese Verfahren haben alle den Nachteil, dass sie langedauernde Prozesse bedingen, die für den Patienten schmerzhaft sind, und mehrere Operationen benötigen. Der Vorteil eines einmaligen, injizierbaren Verfahrens liegt in der erhöhten Akzeptanz durch den Patienten da das Verfahren sehr schonend ausgeführt und leicht ertragen wird.

Ein injizierbares Verfahren zur Kallusdehnung ist aus der WO2012123095 bekannt. Diese bekannte Vorrichtung betrifft eine Granulatmischung zur Regeneration eines Knochens, mittels dreidimensionaler Dehnung zur Kallusdehnung. Die Granulatmischung beinhaltet starres und verformbares Granulat welches nach dem Einspritzen in den Knochendefekt durch die Expansion eines Hydrogels in dem Verformbaren Granulat eine Dehnung auf das umgebende Körpergewebe ausführt. Bei dieser bekannten Vorrichtung ist nachteilig, dass durch die Verwendung eines osmotisch wirkenden Verfahrens, wie z.B. durch Anwendung von Hydrogel, die Bewegung einmalig und irreversibel ist. Nach Erreichen der Endausdehnung wird keine Bewegung mehr ausgeführt. Die Aktivierung der Gewebsdifferenzierung bedingt aber eine wiederholte Bewegung. Auch ist von Nachteil, dass das Spektrum der Amplitude und Geschwindigkeit der Bewegung durch die chemischen Eigenschaften des Hydrogels sehr eingeschränkt sind. Die Anregung der Knochenbildung ist damit nicht zuverlässig und kurzfristig und fällt deutlich kleiner aus als bei in der vorliegenden Erfindung. Ein weiterer Nachteil ist, dass nach Einbringen der Granulatmischung in den Körper die einmalige Bewegung nicht mehr verändert werden kann. Somit ist es nicht möglich auf veränderte Bedingungen die während der Behandlung auftreten zu reagieren, was wichtig ist um die Gewebebildung im Verlauf des Heilungsprozesses optimal an die unterschiedlichen konsekutiven Stadien der Frakturheilung anzupassen, was unterschiedliche Werte der zur Induktion der Differenzierung notwendigen Dehnung benötigt.

Aus der WO2008/043484 ist weiter ein Gerüst zur künstlichen dreidimensionalen Kallusdistraktion eines Knochens bekannt, welches in den Körper eines Patienten eingebracht werden kann. Einen extern angebrachten Apparat kann von aussen her das Gerüst bewegen, welches seinerseits seine Bewegung an die umgebenden Körperzellen des Patienten abgeben kann. Die Nachteile dieser bekannten Vorrichtung sind die folgenden:
(i) das Gerüst weist einen komplizierten Faserverbund mit Zwischenräumen auf, um durch ein Dehnung/Schrumpfung des Gerüstes Kräfte auf das umgebende Gewebe des Patienten einwirken zu können;
(ii) bei Verwendung mehrerer Gerüste wird das einzelne Gerüst nur durch eine Volumenänderung aktiviert, nicht aber die relative Lage der einzelnen Gerüste zueinander.

Hier will die vorliegende Erfindung Abhilfe schaffen. Der Erfindung liegt die Aufgabe zugrunde, eine Vorrichtung zu schaffen, welche auf Grund ihrer einfachen und sicheren Handhabung eine optimale und gezielte dynamische Deformation von Körpergeweben gestattet.

Im Zusammenhang mit der Erfindung werden folgende Definitionen verwendet:

### Dehnung (Formelzeichen ε):

Unter Dehnung wird im Nachfolgenden eine Angabe für die relative Deformation (Verlängerung oder Verkürzung) in verschiedenen Richtungen eines Körpers unter Belastung, beispielsweise durch Krafteinwirkung verstanden. Wenn sich die Abmessung des Körpers vergrössert, wird dies als positive Dehnung bezeichnet, andernfalls als negative Dehnung oder Stauchung.

Kritisches Element der Mechanik in Bezug auf die Knochenheilung ist die Gewebe-Deformation (Dehnung ε im Sinne der Verlängerung und der Verkürzung, in verschiedenen Richtungen). Die Heilung wird einerseits angeregt, wenn die Gewebedehnung grösser als ein Mindestbetrag ist. Bei zu geringer Dehnung wird der Heilungsprozess nicht ausgelöst. Die Überbrückung des Bruchs ist andererseits nur möglich, wenn die vorliegende Dehnung des Gewebe-Elements nicht grösser als die Bruchelongation des reparierenden Gewebes ist. Zwischen diesen beiden Bedingungen, der Induktion und der Toleranz, besteht eine Bandbreite der Dehnung, die Voraussetzung für eine speditive und zuverlässige Heilung ist. Liegt die interfragmentäre Dehnung ausserhalb der erwähnten Bandbreite ist die Heilung gestört. Es entstehen Heilungsverzögerungen oder Pseudoarthrosen. Die Behandlung derartiger Heilungsstörungen bedingt eine Korrektur der Gewebedehnung. Durch Vergrösserung oder Verkleinerung kann die Dehnung in die erwähnte Bandbreite zurückgeführt und damit die Heilung ermöglicht werden.

### Dehnungsfrequenz:

Die Häufigkeit der Gewebsdeformation pro Zeiteinheit, der Begriff Dehnung (strain = dL/L) ist hier richtungsunabhängig (wird aber oft als Gegensatz zu Zusammendrücken verwendet)

### Nicht-invasive Induktion dynamischer Deformation von Körpergewebe:

Nicht invasiv - ohne Gewebstraumatisierung , dynamische Deformation = zeitabhängige Deformation von Körpergewebe. Im Zusammenhang mit der Erfindung soll diese Kombination einen Apparat beschreiben der von aussen angebracht wird ohne körperfremde physische Verbindung durch das Köpergewebe auf die Partikel Einfluss ausübt. Dadurch wird keine Verletzung der Haut und Gewebeschichten verursacht. Dies im Gegensatz zum bekannten Illizarov-Apparat.

### Heterotope Gewebebildung:

### Bildung von Gewebe an Orten wo diese Gewebe entsprechen der Natur nicht vorkommt

Die durch die Erfindung gegenüber dem Stand der Technik erreichten Vorteile sind im Wesentlichen darin zu sehen, dass:
- die Injektion einer Suspension von in Lösung suspendierter - mittels des externen Aktuators aktivierbaren - Partikel gestattet eine äusserst einfache und zielgenaue Applikation;
- durch die von aussen induzierte Bewegung der Partikel wird wiederholt Dehnung auf das umgebende Gewebe ausübt wodurch die Heilung optimiert wird.
- die von aussen aktivierte Bewegung kann in Intensität und Frequenz gesteuert werden, wodurch die Dehnung sich immer im optimalen Bereich der konsekutiven Differenzierungsstadien gehalten werden kann.
- eine heilungsstörende, zu geringe oder zu grosse Dehnung kann vermieden werden
- die Dehnung lässt sich optimal an die biologischen Notwendigkeiten anpassen und es ist jederzeit möglich die Dehnung an den Heilungsfortschritt anzupassen;
- die vorliegende Erfindung ermöglicht die zur Knochen- und Gewebebildung notwendige Gewebedehnung in optimaler Bandbreite von Amplitude und Frequenz von aussen d.h. ohne transkutane Implantat-Verbindung - die einer Infektion Vorschub leisten würde - zu aktivieren
- die Partikel können im Verlauf des Heilungsprozesses vom Körper resorbiert oder unschädlich ins Körpergewebe eingelagert werden,
- die Erfindung kann überall dort angewendet werden, wo durch Korrektur oder Erzeugung der optimalen Dehnung Gewebsdifferenzierung angeregt oder ermöglicht werden muss. Unter anderem ist z.B. die Erzeugung von Weichgewebe oder Knochen für therapeutische oder ästhetische Anwendungen möglich. Bei der Knochenverlängerung nach der lllizarov Methode wird heute in täglichen millimetergrossen Schritten mehrstufig verlängert da nach grösseren Schrittes eine zu geringe Dehnung entstehen würde, die eine zuverlässige Knochenbildung nicht bewirkt. Durch Anwendung der erfindungsgemässen Vorrichtung wird die für die Knochenbildung optimale Dehnung auch bei zentimetergrossen Verlängerungsschritten erreicht. Die Tragzeit der den Patienten störenden externen Fixateure kann durch grosse Einzelschritte der Dehnung wesentlich verkürzt oder vermieden werden.
- bei chirurgischen Eingriffen, die eine Transplantation von autologem (körpereigenen) Knochen bedingen, bewirkt die notwendige Knochenentnahme, z.B. am Becken, länger dauernde störende Schmerzen. Die erfindungsgemässe Vorrichtung kann im Patienten eingesetzt die Knochenbildung erzeugen ohne die erwähnten schmerzhaften Folgen der Knochen-Entnahme.
- die Erfindung erlaubt zuverlässig und schmerzlos, eine für die Gewebedifferenzierung optimale Dehnung in einem Gewebe zu erzeugen, wodurch sich gegenüber den heute üblichen Verfahren eine wesentliche Beschleunigung und höhere Zuverlässigkeit erzielbar ist.
- die erfindungsgemässe Vorrichtung kann dazu dienen an definierten Stellen die Heilung zu fördern. Sie kann aber auch dazu dienen bestimmte Gewebe im Körper herzustellen, die generell für das "tissue engineering" eingesetzt werden.
- die erfindungsgemässe Vorrichtung kann auch für die Knochenverlängerung eingesetzt werden. Sie erlaubt solche Verlängerungen bis zu mehreren Zentimetern in einem einzigen Schritt bei erhaltener Induktion der Gewebsbildung. Dies ist der wesentliche Vorteil gegenüber der bekannten Illizarov Methode, welche nur millimetergrosse, tägliche Verlängerungsschritte gestattet. Mit der erfindungsgemässen Vorrichtung kann somit die Knochenverlängerung in einem oder wenigen Schritten erreicht werden. Der Patient ist kurz nach der Operation wieder voll funktionsfähig und das wochenlange Tragen bewegungsbehindernder externer Fixateur-Konstruktionen kann durch die erfindungsgemässe Vorrichtung überflüssig gemacht werden.
- die erfindungsgemässe Vorrichtung kann in allen Körperteilen eingesetzt werden.

Weitere vorteilhafte Ausgestaltungen der Erfindung können wie folgt kommentiert werden:
Bei einer besonderen Ausführungsform der Erfindung können die Partikel Körperzellen umfassen.

Bei einer weiteren Ausführungsform sind die Partikel nicht-porös.

Die Partikel können metallisch, magnetisch, magnetisierbar, elektrisch ladbar, massenreich, oder thermisch reaktiv seine. Vorzugsweise umfassen die Partikel eine oder mehrere der folgenden Substanzen:
Platin, Titan, Stahl, Kohlestoff-Stahl, kristalline Legierungen auf Basis Eisen, Nickel, Kobalt, amorphe oder nano-kristalline Legierungen auf Basis Eisen, Nickel, Kobalt; Weichferrite, Kobalt Samarium, Neodym Eisen-Bor, AlNiCo, Hartferrite, martensitische Stähle, Memory Alloys.

Die Partikel können auch elektrisch leitende Materialien umfassen, die durch eine isolierende Aussenhaut ein zur Umgebung unterschiedliches Ruhepotential aufweisen. Bei einer weiteren Ausführungsform umfassen die Partikel ein bioresorbierbares Material.

Bei einer weiteren Ausführungsform sind die Partikel mindestens in ihrem Kern homogen aufgebaut.

Die Partikel können auch mit einer biokompatiblen Aussenhaut versehen sein, welche geeignet ist das Anwachsen von Körperzellen zu fördern.

Der durchschnittliche Durchmesser der einzelnen Partikel ist zweckmässigerweise grösser als 5 µm. Der durchschnittliche Durchmesser der einzelnen Partikel ist zweckmässigerweise kleiner als 5 mm, vorzugsweise kleiner als 50 µm.

Das durchschnittliche Volumen der einzelnen Partikel ist zweckmässigerweise grösser als 125 µm³, vorzugsweise grösser als 1000 µm³ . Das durchschnittliche Volumen der einzelnen Partikel ist zweckmässigerweise kleiner 100 mm³, vorzugsweise kleiner als 1 mm³.

Bei einer weiteren Ausführungsform weisen die Partikel keine faserige Struktur auf.

Die körperverträgliche Flüssigkeit kann knochenbildende Substanzen und/oder Gewebebestandteile und/oder Stammzellen enthalten.

Pro mm³ körperverträgliche Flüssigkeit sind maximal 10⁶ Partikel, vorzugsweise maximal 10⁵ Partikel enthalten. Das Verhältnis zwischen dem Gesamtvolumen ΣVol_{P} der Partikel und dem Volumen der Flüssigkeit Vol_{F} beträgt zweckmässigerweise maximal 10:1, vorzugsweise maximal 2:1. Das Verhältnis zwischen dem Gesamtvolumen ΣVol_{P} der Partikel und dem Volumen der Flüssigkeit Vol_{F} beträgt zweckmässigerweise minimal 1:1000, vorzugsweise minimal 1:10.

Bei einer speziellen Ausführungsform der Erfindung umfassen die suspendierten Partikel zwei oder mehr unterschiedliche Typen, die so ausgeführt sind, dass durch den Aktuator angeregt, eine Bewegung zwischen den unterschiedlichen Typen von Partikeln ausgeführt wird. Dies bewirkt Dehnung und Kompression der sie umgebenden Zellen oder Gewebe. Diese Aktivierung kann magnetisch, elektrisch, nano- oder quantentechnologisch, auf Massenträgheit beruhend, thermisch oder eine andere von aussen angebrachte gewebedurchdringende nicht invasive Art ausgeführt werden.

Die Partikel sind biokompatibel. Die Partikel können auch mit Kalzium-Phosphat angereichert sein.

Bei einer besonderen Ausführungsform kann d er Aktuator elektromagnetische Wellen emittieren.

Der Aktuator kann zwei Magnetspulen umfassen, welche alternierend Strom führen und damit magnetisierbare Partikel bewegen können.

Der Aktuator kann auch eine Vibrations-Quelle umfassen, welche vorzugsweise in einem Frequenzbereich von 1/10 - 1/1800 Hz (s⁻¹) emittiert.

Beschrieben wird auch ein Verfahren zur nicht-invasiven Induktion dynamischer Deformation von Körpergewebe zur Differenzierung von Gewebezellen, welches durch folgende Schritte gekennzeichnet ist:
a) Injektion einer körperverträglichen Flüssigkeit, in welcher volumenkonstante Partikel suspendiert sind, in das zu deformierende Körpergewebe;
b) Aktivierung der Partikel mittels eines externen Aktuators ohne mechanische Verbindung zwischen Aktuator und Partikel, wobei
c) die Aktivierung magnetisch, elektrisch,, vibratorisch, oder thermisch erfolgt; so dass
d) eine Bewegung der Partikel erzeugt wird, die im Körpergewebe zu Deformationen führt, welche die reparative Differenzierung von Körperzellen anregt.

Bei diesem Verfahren diffundiert die Suspension der Partikel in das zu behandelnde Körpergewebe, wobei letzteres durch die externe Aktivierung deformiert wird. Dies erfolgt entweder durch direkte Kraftübertragung, wenn die Körperzellen an die Partikel angewachsen sind oder über eine Verdrängung der Körperzellen.

Bei einer besonderen Ausführungsform des Verfahrens kann die körperverträgliche Flüssigkeit zusätzlich zu den Partikeln Körperzellen enthalten, welche durch die externe Aktivierung der Partikel deformiert werden.

Bei einer weiteren Ausführungsform des Verfahrens werden die Partikel mittels der externen Aktivierung einheitlich ausgerichtet. Bei einer anderen Ausführungsform des Verfahrens führen die Partikel mittels der externen Aktivierung eine Translationsbewegung aus.

Das Verfahren kann auch eine heterotope Gewebebildung ermöglichen.

Bei einer besonderen Ausführungsform des Verfahrens wird der Aktuator mindestens eine Stunde, vorzugsweise mindestens 3 Stunden lang betätigt. Zweckmässigerweise wird der Aktuator höchstens 6 Monate, vorzugsweise höchstens 1 Monat lang betätigt.

Die Ausrichtung oder Formänderung der Partikel erfolgt zweckmässigerweise mindestens alle 2 Stunden, vorzugsweise mindestens alle 15 Minuten. Bei einer anderen Ausführungsform des Verfahrens erfolgt die Ausrichtung oder Formänderung der Partikel höchstens alle Sekunden, vorzugsweise höchstens alle 10 Sekunden.

Die Erfindung und Weiterbildungen der Erfindung werden im Folgenden anhand der teilweise schematischen Darstellungen noch näher erläutert.

### Es zeigen:

Beispiel einer Chirurgischen Osteosynthese mittels eines Implantats das dem Stand der Technik entspricht und der Vorrichtung zur nicht-invasiven Induktion dynamischer Deformation von Körpergewebe.

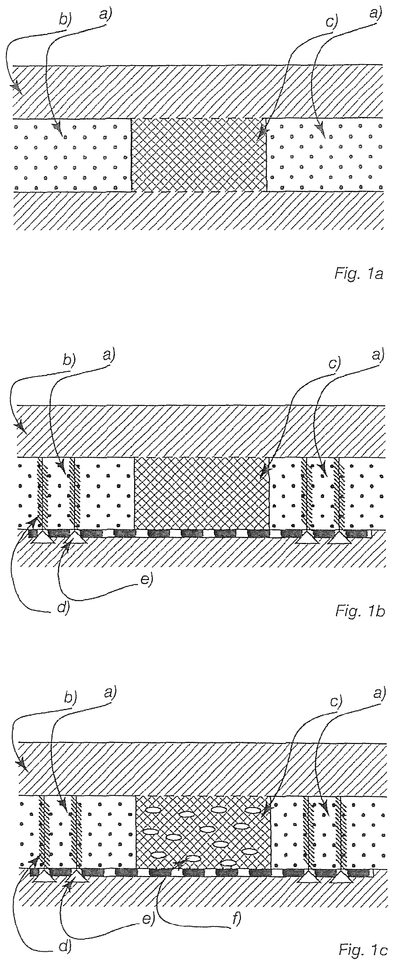

Fig. 1a: Der Knochen (a) im Körper (b) ist gebrochen, die Grösse des Defekts (c) verhindert bei einer Operation mittels Implantaten die dem Stand der Technik entsprechen, die Heilung des Knochens durch fehlende knöcherne Überbrückung.
Fig. 1b: Die Knochenfragmente werden mittels eines Implantats (e), das dem Stand der Technik entspricht mit oder ohne Schrauben (d) kraftschlüssig so verbunden, dass wenig bis keine Bewegung zwischen den Knochenfragmenten (a) entsteht.
Fig. 1c: Die Lösung suspendierter Partikel (f) die Körperzellen enthalten kann wird in den Defekt eingebracht. Dieses kann zum Beispiel durch Einspritzen erfolgen.

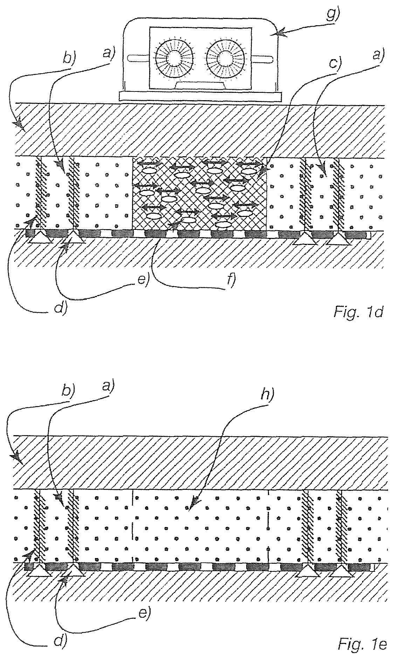

Fig. 1d: Der Aktuator (g) wird extern angebracht. Bei dessen Aktivierung entstehen in der Lösung suspendierter Partikel, die Körperzellen enthalten kann, gegenseitige Bewegungen und somit Dehnung die sich auf die Körperzellen und/oder das Körpergewebe überträgt. Die Körperzellen und/oder das Körpergewebe beginnt sich zu Differenzieren. Im vorliegenden Fall bildet sich in verschiedenen Differenzierungsstufen Knochen-gewebe. Ein wichtiger Vorteil der vorliegenden Erfindung ist, dass die Induktion optimal an den individuellen, fortschreitenden Heilungsprozess angepasst werden kann.
Fig. 1e: Der Knochen hat über eine Knochenbrücke (h) die beiden Knochenfragmente wieder verbunden.
   Beispiel einer Knochenverlängerung durch Trennung und chirurgischen Osteosynthese mittels eines Implantats das dem Stand der Technik entspricht und der Vorrichtung zur nichtinvasiven Induktion dynamischer Deformation von Körpergewebe.

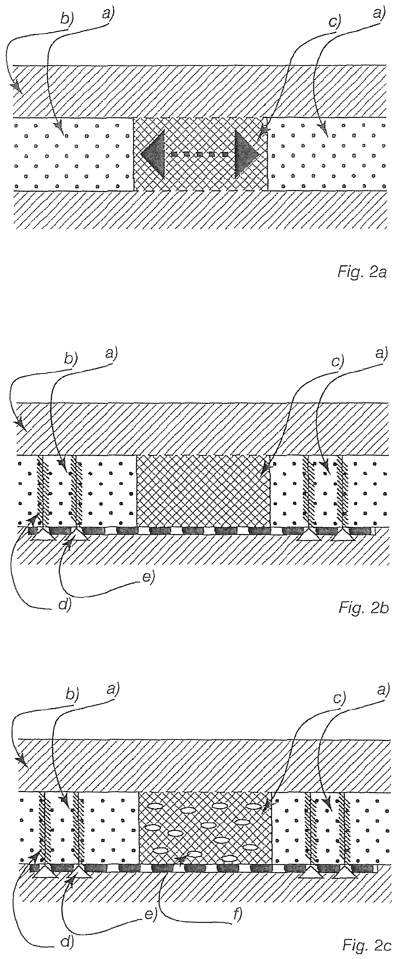

Fig. 2a: Der Knochen (a) im Körper (b) wird chirurgisch getrennt. Die Beiden Knochenfragmente werden auseinandergezogen und in gewünschter Position durch ein Implantat das dem Stand der Technik entspricht fixiert. Die Grösse des Defekts (c) verhindert bei einer einstufigen Operation die Heilung des Knochens durch fehlende knöcherne Überbrückung.
Fig. 2b: Die Knochenfragmente werden mittels eines Implantats das dem Stand der Technik entspricht mit oder ohne Schrauben (d) kraftschlüssig so verbunden, dass wenig bis keine Bewegung zwischen den Knochenfragmenten entsteht.
Fig. 2c: Die Lösung suspendierter Partikel die Körperzellen enthalten kann wird in den Defekt eingebracht. Dieses kann zum Beispiel durch Einspritzen erfolgen.

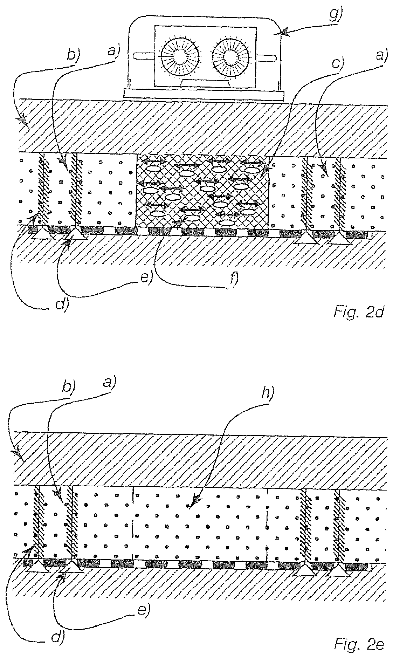

Fig. 2d: Der Aktuator (g) wird extern angebracht. Bei dessen Aktivierung entstehen in der Lösung suspendierter Partikel, die Körperzellen enthalten kann, gegenseitige Bewegungen und somit Dehnung die sich auf die Körperzellen und/oder das Körpergewebe überträgt. Die Körperzellen und/oder das Körpergewebe beginnt sich zu Differenzieren. Im vorliegenden Fall bildet sich in verschiedenen Differenzierungsstufen Knochengewebe. Ein wichtiger Vorteil der vorliegenden Erfindung ist, dass die Induktion optimal an den individuellen, fortschreitenden Heilungsprozess angepasst werden kann.
Fig. 2e: Der Knochen hat die beiden Knochenfragmente wieder über eine Knochenbrücke (h) verbunden. Der Knochen wurde um die Breite des eingeführten Defekts erweitert. So kann eine Knochenverlängerung von bis zu 50 mm in einem Schritt erfolgen.
   Beispiel einer Gewebeneubildung durch induzierte Differenzierung in Patienten durch die Vorrichtung zur nicht-invasiven Induktion dynamischer Deformation von Körpergewebe.

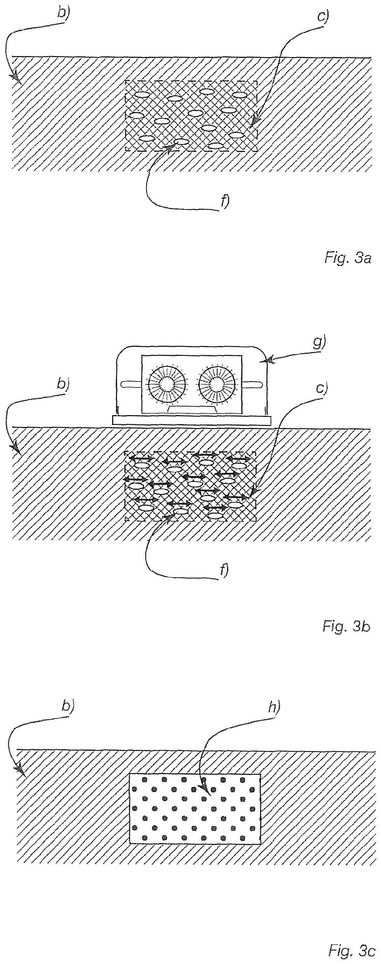

Fig. 3a: Die Lösung suspendierter Partikel die Körperzellen enthalten kann wird in Muskelgewebe des Patienten eingebracht. Dieses kann zum Beispiel durch Einspritzen erfolgen.
Fig. 3b: Der Aktuator wird extern angebracht. Bei dessen Aktivierung entstehen in der Lösung suspendierter Partikel die Körperzellen enthalten kann eine Bewegung und somit eine Dehnung die sich auf die Körperzellen und/oder das Körpergewebe überträgt. Die Körperzellen und/oder das Körpergewebe beginnt sich zu Differenzieren.
Fig. 3c: Je nach Dauer und Stärke wird unterschiedliches Gewebe gebildet, so zum Beispiel Bindegewebe, Knorpel oder Knochen.
   Beispiel von Knochenwiederherstellung bei Knochendefekt, wie zum Beispiel durch Knochenschwund oder als Unfallfolge, durch die Vorrichtung zur nicht-invasiven Induktion dynamischer Deformation von Körpergewebe.

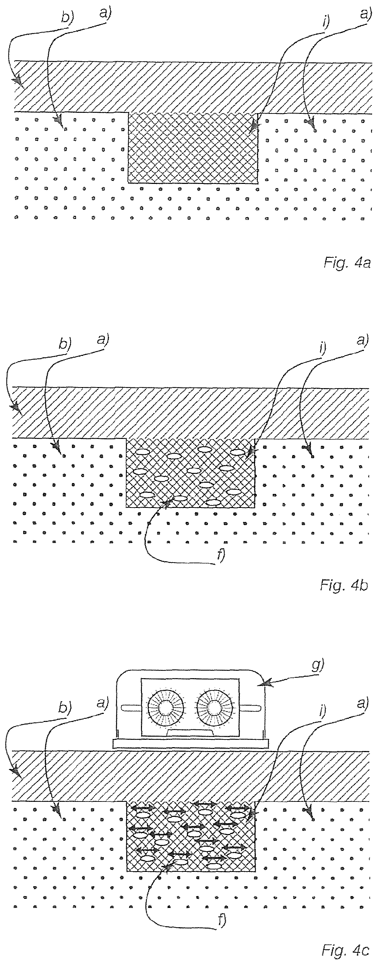

Fig. 4a/b: Die Lösung suspendierter Partikel die Körperzellen enthalten kann wird an den Ort des Knochendefekts eingebracht. Dieses kann zum Beispiel durch Einspritzen erfolgen.
Fig. 4c: Der Aktuator wird extern angebracht. Bei dessen Aktivierung entstehen in der Lösung suspendierter Partikel die Körperzellen enthalten kann eine Bewegung und somit eine Dehnung die sich auf die Körperzellen und/oder das Körpergewebe überträgt. Die Körperzellen und/oder das Körpergewebe beginnt sich zu Differenzieren.
Fig. 4d: Der Knochen wird wiederhergestellt.
   Beispiel von anderen Ausführungsarten der Partikel die in Kombination mit den zuvor genannten Beispielen angewendet werden können.
Fig. 5a: Die Lösung suspendierter Partikel die Körperzellen enthalten kann wird eingebracht. Dieses kann zum Beispiel durch Einspritzen erfolgen. Die Partikel sind hier als Stäbchen geformt.
Fig. 5b: Der Aktuator wird extern angebracht. Bei dessen Aktivierung deformieren sich die Stäbchen. Dadurch entsteht in der Lösung suspendierter Partikel die Körperzellen enthalten kann eine Bewegung und somit eine Dehnung die sich auf die Körperzellen und/oder das Körpergewebe überträgt.
Fig. 5c: Der Aktuator kann auch die Partikel so steuern, dass diese wieder in die Grundstellung übergehen. Der gesamte Vorgang kann so wiederholt werden, dass es in einem für die Körperzellen und/oder das Körpergewebe optimalen Frequenzbereich und Intensität wiederholt wird damit die optimale/gewünschte Differenzierung erreicht wird.
Fig. 5d: Die Körperzellen und/oder das Körpergewebe beginnt sich zu Differenzieren. Die Partikel können vom Körper resorbiert werden oder werden in das entstehende Körpergewebe eingebaut. Der Vorteil der Vorrichtung zur Induktion dynamischer Deformation von Körpergewebe ist zum einen, dass die Differenzierung grundsätzlich eingeleitet werden kann und zum zweiten, dass die Differenzierung in Tempo, und resultierendes Gewebe gesteuert werden kann.

### Beispiel 1 (Pseudoarthrose Behandlung):

Die Patientin leidet nach einem Verkehrsunfall an einer nicht heilenden Knochenfraktur des mittleren Oberschenkelknochens. Die Fraktur besteht aus vielen Teilen. Nach einer Reihe nicht erfolgreicher, chirurgischer Osteosynthesen bleiben die Knochenteile getrennt. Zum Teil hat sich der Knochen abgebaut. Ein Grund für die Nicht-Heilung ist die fehlende Stimulierung durch einen zu geringen Dehnungsreiz, verursacht durch die geometrische Komplexität des Bruchs. Die traditionelle Fixierung der Knochenteile erlaubt nicht eine Stimulierung für die verschiedenen Knochenzwischenräume.

In diesem Fall wird die stabile traditionelle Verbindung im Patienten belassen. In die nicht heilenden Knochenzwischenräume wird eine mit magnetisierten oder magnetisch aktiven Neodym-Partikel versetzte Ringerlösung mit körpereigenen Stammzellen injiziert.

Die Neodym-Partikel sind mit einem Kunststoff umschlossen, so dass diese körperverträglich sind. Die Oberfläche der Partikel ist derart gestaltet, dass die Bewegung zweckentsprechend übertragen wird. Das durchschnittliche Volumen der verwendeten Neodym-Partikel betrug 1'000'000 µm³.

Eine Reihe weiterer Versuche wurde mit Neodym-Partikeln durchgeführt, welche ein durchschnittliches Volumen im Bereich von 1250 - 4'250'000 µm³ aufwiesen.

Die injizierte Ringer-Lösung enthielt 50'000 Partikel pro mm³. Eine Reihe weiterer Versuche wurde mit körpereigener Flüssigkeit (statt der Ringer-Lösung) durchgeführt, mit einer Partikeldichte im Bereich von 250 - 200'000 Stück pro mm³.

Das Volumen der injizierten Flüssigkeit entsprach dem Zwischenraum der in dem vorliegenden Knochenbruch entstanden war, im konkreten Fall 5cl.

Nach erfolgter Injektion der Lösung mit den Partikeln wurde eine Aktuator für diese Partikel extern angesetzt. Dieser bestand aus einer Befestigungskomponente und einer technischen Komponente. Die Befestigungskomponente war als Manschette aus textilem Material ausgebildet. Die technische Komponente bestand aus einem Steuerungsmodul, einem Induktionsmodul und einer Batterie. Das Induktionsmodul bestand aus zwei beweglichen Spulen die bei der Aktivierung vor- und zurückbewegt wurden.

Der Aktuator wurde auf eine Frequenz von 6 Induktionen pro Stunde (1/600 sec¹(Hz)) eingestellt und aktiviert.

Die Heilung wurde radiologisch überwacht, wobei die Frequenz und Amplitude an den Heilungsfortschritt angepasst wurde. Die Grösse der Amplitude wurde dadurch bestimmt, dass die durch die Partikel übertragene Gewebsdehnung in der Grösse von 100-2% entstand. Die Amplitude wurde während des Heilungsfortschritts reduziert.

Nach 5 Wochen war die Heilung abgeschlossen, die Knochen waren solide verbunden und tragfähig. Die biochemisch inerten Partikel verblieben bis zur Ausscheidung im Körper oder wurden in den Knochen eingebaut. Der Aktuator wurde nach erfolgter Heilung entfernt.

### Beispiel 2 (körpereigene Knochenproduktion):

Der Patient litt unter einem Knochendefekt nach einem Trauma in der Folge eines Sportunfalls. Als Knochenersatz wurde Spongiosa benötigt. Die Gewinnung der Spongiosa stellte einen wesentlichen zusätzlichen chirurgischen Eingriff dar, der längere Zeit starke Schmerzen verursachte. In diesem Fall wurde durch die Erfindung der Knochendefekt knöchern aufgefüllt, indem durch Injektion eine mit thermisch aktivierbaren Partikeln angereicherte Stammzellenlösung (MSC) in den Knochendefekt eingespritzt wurde.

Die thermisch aktivierten Partikel waren so aufgebaut, dass durch eine von aussen eingebrachte Temperaturänderung sich eine Spannung in den Partikeln aufbaute, die beim Erreichen einer Grenzspannung, entladen und eine pulsförmige Bewegung des Partikels erzeugt wurde. Nach der Entspannung und dem Erreichen der Körpertemperatur waren die Partikel wieder bereit für einen weiteren Zyklus. Die verwendeten Partikel waren von länglicher Form, und aus mehreren Lagen verschiedener thermisch reagierender Metalle aufgebaut (in einem weiteren Versuch wurde dazu ein Memory Alloy verwendet). Aussen waren die Partikel mit einer elastischen, körperverträglichen Kunststoffschutzschicht überzogen. Die Oberfläche war so gestaltet, dass die Bewegung zweckentsprechend übertragen wurde.

Die durchschnittliche Grösse der Partikel betrug 0,1 mm.

Die Anzahl Partikel pro Volumeneinheit der injizierte Lösung betrug 10 Stück pro mm³.

Der in diesem Versuch verwendete thermische Aktuator bestand aus einer Befestigungskomponente und einer technischen Komponente. Die Befestigungskomponente war als textile Manschette ausgeführt. Die technische Komponente bestand aus einem Steuerungsmodul, einem Induktionsmodul und einer Batterie. Das Induktionsmodul war eine Wärmequelle die auf thermischer Induktion basiert und die Partikel in der Lösung in dem nahe an der Oberfläche liegenden Knochendefekt so aufwärmt, dass diese die vorgesehene Bewegung durchführen konnten, ohne das umgebende Gewebe zu schädigen.
wird von aussen angebracht. Die am Aktuator eingestellt Frequenz betrug 2-mal pro Stunde also auf 1/1800 sec⁻¹(Hz) eingestellt.

Die Leistung des Aktuators wurde so eingestellt, dass die Partikel zweckentsprechend aktiviert wurde, ohne das umgebende Gewebe zu schädigen. Die Partikel wurde soweit erwärmt, dass die Bewegung nicht gewebeschädigend war.

Die Partikel übten durch die thermische Aktivierung eine Dehnung auf die in der Lösung enthaltenen MSC aus. Diese begannen sich zu differenzieren und bildeten innerhalb von circa 6 Wochen körpereigenen Spongiosa-Knochen auf schmerzfreie Art.

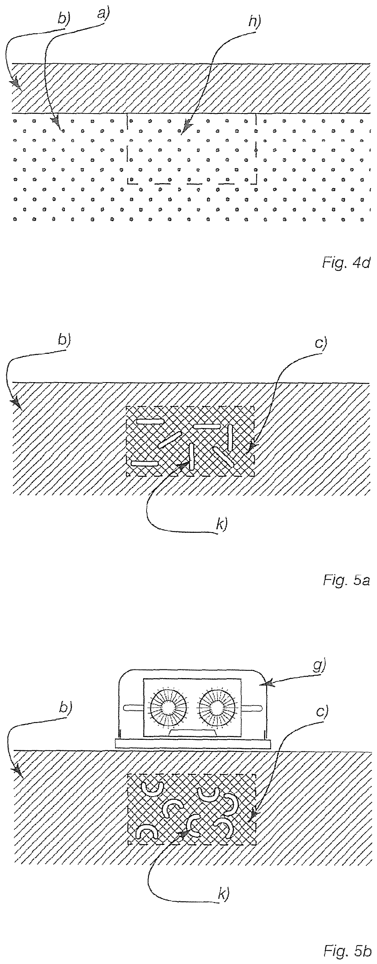

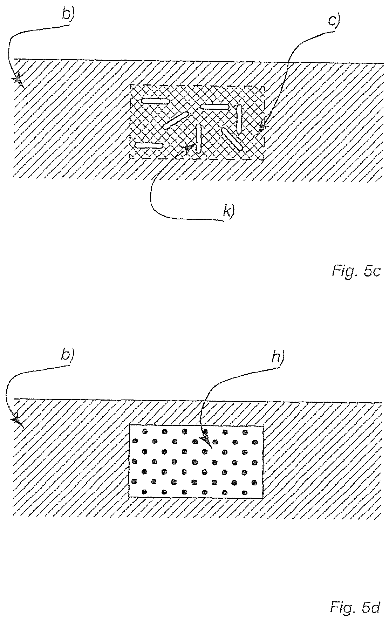

Bei der Aktivierung der Partikel bleib deren Volumen konstant, lediglich ihre Form änderte sich von gekurvt zu gerade, wie in den Figuren 5a, b, und c dargestellt.

### Beispiel 3 (Knochenverlängerung)

Die Patientin litt seit der Adoleszenz an einer einseitigen Verkürzung des Unterschenkels von 20 mm durch einen Wachstumsfehler. Um absehbaren Haltungsschäden entgegenzuwirken sollte die Länge der betroffenen Gliedmassen angepasst werden. Hierzu wurden die beiden Unterschenkelknochen des verkürzten Beines in einem chirurgischen Eingriff an geeigneter Stelle getrennt
und mittels traditionellen orthopädischen Fixierungsmetoden so stabilisiert, dass beide Beine gleich lang waren. Es entstand ein Segment, in welchem der Knochen fehlte. In dieses Segment wurde eine Lösung mit Partikeln injiziert die eine grosse spezifische Masse hatten. Die Partikel bestanden aus einem Kern von hoher spezifischer Masse und einer biokompatiblen Hülle. Die Oberfläche war so gestaltet, dass die Bewegung durch Haftung des Gewebes zweckentsprechend übertragen wurde. Der Kern der Partikel bestand aus Platin, die Hülle aus geätztem Titan. Die mittlere Grösser der Partikel betrug 0,15 mm. Die Anzahl der Partikel pro Volumeneinheit der injizierten Lösung betrug 8 Stück pro mm³

Die Menge an Injizierter Lösung betrug 6 ml.

Der auf diese Partikel abgestimmte Aktuator wurde von aussen an das Bein angebracht und 3 Mal pro Stunde aktiviert. (1/1200 Hz)
Der Aktuator funktionierte nach dem Prinzip der Resonanz und bestand aus einer Befestigungskomponente und einer technischen Komponente. Die Befestigungskomponente war in dieser Anwendung eine Manschette aus Kunststoff. Die technische Komponente bestand aus einem Steuerungsmodul, einem Induktionsmodul und einer Batterie. Das Induktionsmodul war eine Bewegungsquelle die auf Rotationsunwucht basiert und die Partikel in der Lösung im Knochendefekt auf Grund Ihrer Massenträgheit gegenüber dem umgebenden Gewebe bewegte.

Die Heilung wurde radiologisch überwacht. Die Frequenz und Amplitude an den Heilungsfortschritt angepasst.

Die Gewebezellen, auf die ein Dehnungsreiz wirkte, begannen sich zu differenzieren, beginnend mit einer zellhaltiger Flüssigkeit. Diese wandelt sich etwa im Wochentakt in Granulationsgewebe, zu belastbaren Bindegewebe um.

Nach 6 Wochen war die Heilung abgeschlossen, die Knochen waren solide verbunden und tragfähig. Die biochemisch inerten Partikel verblieben bis zur Ausscheidung im Körper oder wurden in den Knochen eingebaut. Der Aktuator wurde nach erfolgter Heilung entfernt.

### Beispiel 4 (Tissue Engineering).

Der Patient litt an einer sportbedingten Funktionsstörung im linken Schultergelenk die schmerzhaft war. Hierfür sollte eine neue Gelenkskapsel hergestellt werden. Diese wurde aus einer Bindegewebsmembran hergestellt. Die Bindegewebsmembran wurde mit dem erfindungsgemässen Verfahren im Körper produziert.

Dazu wurde in der Bauchregion chirurgisch subkutan eine flache Tasche hergestellt. In diese Tasche wurde eine Lösung mit Partikeln injiziert, welche eine grosse spezifische Masse aufwiesen. Die Partikel bestanden aus einem Kern von hoher spezifischer Masse und einer biokompatiblen Hülle. Die Oberfläche war so gestaltet, dass die Bewegung durch Haftung des Gewebes zweckentsprechend übertragen wurde. Der Kern der Partikel bestand aus Platin, die Hülle aus geätztem Titan.

Für diese Anwendung weisen die Partikel eine mittlere Länge von 0,2 mm auf.

Die Anzahl der Partikel pro Volumeneinheit betrug 7 Stück pro mm³
Das injizierte Volumen betrug 8 ml
Der auf diese Partikel abgestimmte Aktuator funktionierte nach dem Prinzip der Resonanz und bestand aus einer Befestigungskomponente und einer technischen Komponente. Die Befestigungskomponente war in dieser Anwendung eine Manschette aus Kunststoff. Die technische Komponente bestand aus einem Steuerungsmodul, einem Induktionsmodul und einer Batterie. Das Induktionsmodul war eine Bewegungsquelle die auf Rotationsunwucht basiert und die Partikel in der Lösung im Knochendefekt auf Grund Ihrer Massenträgheit gegenüber dem umgebenden Gewebe bewegte.

Der Aktuator wurde von aussen an den Bauch angebracht und 3 Mal pro Stunde aktiviert. (1/1200 Hz)
Die Heilung wurde radiologisch überwacht. Die Frequenz und Amplitude an den Heilungsfortschritt angepasst. Nach 3 Wochen hatte sich in der subkutanen Tasche die Bindegewebsmembran gebildet. Dieses Gewebe wurde in einem chirurgischen Eingriff entnommen und diente zum Ersatz der defekten Gewebskapsel. Die biochemisch inerten Partikel verblieben bis zur Ausscheidung im Körper oder wurden in das Gewebe eingebaut. Der Aktuator wurde nach erfolgter Heilung entfernt.

## Patentansprüche

1. Vorrichtung zur nicht-invasiven Induktion dynamischer Deformation von Körpergewebe zur Differenzierung von Gewebezellen, welche folgende Komponenten umfasst:
(i) eine Suspension von in Lösung suspendierter Partikel; und
(ii) einen externen Aktuator,
wobei
a) die Partikel metallisch, magnetisch, magnetisierbar, elektrisch ladbar oder thermisch reaktiv sind;
b) die Partikel biokompatibel sind;
c) der durchschnittliche Durchmesser der einzelnen Partikel grösser als 5 µm und kleiner als 5 mm ist;
d) das durchschnittliche Volumen der einzelnen Partikel grösser als 125 µm³ und kleiner als 100 mm³ ist;
e) pro mm³ körperverträgliche Flüssigkeit, welche der Suspension zugrunde liegt, maximal 10⁶ Partikel enthalten sind; und
f) der externe Aktuator eine Vibrationsquelle oder eine Wärmequelle umfasst oder elektromagnetische Wellen emittieren oder ein elektrisches Feld erzeugen kann, um die suspendierten Partikel vibratorisch, thermisch, magnetisch oder elektrisch zu erregen.

2. Vorrichtung nach Anspruch 1, **dadurch gekennzeichnet, dass** die Suspension Körperzellen umfasst.

3. Vorrichtung nach Anspruch 1 oder 2, **dadurch gekennzeichnet, dass** die Partikel nicht-porös sind.

4. Vorrichtung nach einem der Ansprüche 1 bis 3, **dadurch gekennzeichnet, dass** die Partikel eine oder mehrere der folgenden Substanzen umfassen:
Platin, Titan, Stahl, Kohlestoff-Stahl, kristalline Legierungen auf Basis Eisen, Nickel, Kobalt, amorphe oder nano-kristalline Legierungen auf Basis Eisen, Nickel, Kobalt; Weichferrite, Kobalt Samarium, Neodym Eisen-Bor, AlNiCo, Hartferrite, martensitische Stähle, Memory Alloys.

5. Vorrichtung nach einem der Ansprüche 1 bis 4, **dadurch gekennzeichnet, dass** die Partikel elektrisch leitende Materialien umfassen, die durch eine isolierende Aussenhaut ein zur Umgebung unterschiedliches Ruhepotential aufweisen.

6. Vorrichtung nach einem der Ansprüche 1 bis 5, **dadurch gekennzeichnet, dass** die Partikel ein bioresorbierbares Material umfassen.

7. Vorrichtung nach einem der Ansprüche 1 bis 6, **dadurch gekennzeichnet, dass** die Partikel mindestens in ihrem Kern homogen aufgebaut sind.

8. Vorrichtung nach einem der Ansprüche 1 bis 7, **dadurch gekennzeichnet, dass** der durchschnittliche Durchmesser der einzelnen Partikel kleiner als 50 µm ist.

9. Vorrichtung nach einem der Ansprüche 1 bis 8, **dadurch gekennzeichnet, dass** das durchschnittliche Volumen der einzelnen Partikel grösser als 1000 µm³ ist.

10. Vorrichtung nach einem der Ansprüche 1 bis 9, , **dadurch gekennzeichnet, dass** das durchschnittliche Volumen der einzelnen Partikel kleiner als 1 mm³ ist.

11. Vorrichtung nach einem der Ansprüche 1 bis 11, **dadurch gekennzeichnet, dass** die körperverträgliche Flüssigkeit knochenbildende Substanzen und/oder Gewebebestandteile und/oder Stammzellen enthält.

## Claims

1. A device for non-invasively inducing dynamic deformation of body tissue for the differentiation of tissue cells, comprising the following components:
(i) a suspension of particle suspended in solution; and
(ii) an external actuator,
wherein
a) the particles are metallic, magnetic, magnetizable, electrically chargeable or thermally reactive;
b) the particles are biocompatible;
c) the average diameter of the individual particles is larger than 5 um and smaller than 5 mm;
d) the average volume of the individual particles is larger than 125 µm³ and smaller than 100 mm³;
e) 1 mm³ biocompatible fluid on which the suspension is based contains not more than 10⁶ particles; and
f) the external actuator comprises a vibration source or a heat source or can emit electromagnetic waves or generate an electric field in order to vibrationally, thermally, magnetically or electrically excite the suspended particles.

2. The device according to claim 1, **characterized in that** the suspension comprises somatic cells.

3. The device according to claim 1 or 2, **characterized in that** the particles are non-porous.

4. The device according to any one of claims 1 to 3, **characterized in that** the particles comprise one or more of the following substances:
platinum, titanium, steel, carbon steel, crystalline alloys based on iron, nickel, cobalt, amorphous or nano-crystalline alloys based on iron, nickel, cobalt; soft ferrites, cobalt samarium, neodymium iron-boron, AlNiCo, hard ferrites, martensitic steels, memory alloys.

5. The device according to any one of claims 1 to 4, **characterized in that** the particles comprise electric conductive materials having a different rest potential to the vicinity through an isolating outer skin.

6. The device according to any one of the claims 1 to 5, **characterized in that** the particles comprise a bioresorbable material.

7. The device according to any one of claims 1 to 6, **characterized in that** the particles are structured homogeneously at least in their core.

8. The device according to any one of claims 1 to 7, **characterized in that** the average diameter of the individual particles is smaller than 50 µm.

9. The device according to any one of claims 1 to 8, **characterized in that** the average volume of the individual particles is larger than 1000 µm³.

10. The device according to any one of claims 1 to 9, **characterized in that** the average volume of the individual particles is smaller than 1 mm³.

11. The device according to any one of claims 1 to 11, **characterized in that** the biocompatible fluid contains boneforming substances and/or tissue components and/or stem cells.

## Revendications

1. Dispositif destiné à l'induction non invasive d'une déformation dynamique de tissus corporels pour la différenciation de cellules tissulaires, lequel comprend les composants suivants :
(i) une suspension de particules en suspension dans une solution ; et
(ii) un actionneur externe,
dans lequel
a) les particules sont métalliques, magnétiques, magnétisables, électriquement chargeables ou thermiquement réactives ;
b) les particules sont biocompatibles ;
c) le diamètre moyen des particules individuelles est supérieur à 5 µm et inférieur à 5 mm ;
d) le volume moyen des particules individuelles est supérieur à 125 µm³ et inférieur à 100 mm³ ;
e) chaque mm³ de liquide physiologiquement compatible, lequel est la base de la suspension, contient un maximum de 10⁶ particules ; et
f) l'actionneur externe comprend une source de vibrations ou une source de chaleur ou peut émettre des ondes électromagnétiques ou générer un champ électrique, afin d'exciter de manière vibratoire, thermique, magnétique ou électrique les particules en suspension.

2. Dispositif selon la revendication 1, **caractérisé en ce que** la suspension comprend des cellules somatiques.

3. Dispositif selon la revendication 1 ou 2, **caractérisé en ce que** les particules sont non poreuses.

4. Dispositif selon l'une des revendications 1 à 3, **caractérisé en ce que** les particules comprennent une ou plusieurs des substances suivantes :
platine, titane, acier, acier au carbone, alliages cristallins à base de fer, de nickel, de cobalt, alliages amorphes ou nanocristallins à base de fer, de nickel, de cobalt ; ferrites douces, cobalt-samarium, néodyme-fer-bore, AlNiCo, ferrites dures, aciers martensitiques, alliages à mémoire de forme.

5. Dispositif selon l'une des revendications 1 à 4, **caractérisé en ce que** les particules comprennent des matériaux électriquement conducteurs, lesquels présentent un potentiel de repos différent de l'environnement de par une membrane extérieure isolante.

6. Dispositif selon l'une des revendications 1 à 5, **caractérisé en ce que** les particules comprennent un matériau biorésorbable.

7. Dispositif selon l'une des revendications 1 à 6, **caractérisé en ce que** les particules sont réalisées de manière homogène au moins dans leur noyau.

8. Dispositif selon l'une des revendications 1 à 7, **caractérisé en ce que** le diamètre moyen des particules individuelles est inférieur à 50 µm.

9. Dispositif selon l'une des revendications 1 à 8, **caractérisé en ce que** le volume moyen des particules individuelles est supérieur à 1000 µm³.

10. Dispositif selon l'une des revendications 1 à 9, **caractérisé en ce que** le volume moyen des particules individuelles est inférieur à 1 mm³.

11. Dispositif selon l'une des revendications 1 à 11, **caractérisé en ce que** le liquide physiologiquement compatible contient des substances ostéogènes et/ou des éléments de tissus et/ou des cellules souches.
